# EUROPEAN PATENT SPECIFICATION

(11) **EP 4 161 483 B1**
(45) Date of publication and mention of the grant of the patent: **15.07.2026**
(21) Application number: 21728578.2
(22) Date of filing: 28.05.2021
(51) Int. Cl.: A61K 8/90, A61K 8/34, A61Q 19/00, A61K 8/31, A61K 8/891, A61K 8/37

(54) **COOLING PERSONAL CARE COMPOSITION COMPRISING A POLYOL AND A POLYOXYEHTYLENE-POLYOXYPROPYLENE BLOCK COPOLYMER**
KÜHLENDE KÖRPERPFLEGEZUSAMMENSETZUNG ENTHALTEND EIN POLYOL UND EIN POLYOXYEHTYLEN-POLYOXYPROPYLEN BLOCK COPOLYMER
COMPOSITION DE SOINS PERSONNELS REFROIDISSANTE COMPRENANT UN POLYOL ET UN COPOLYMÈRE À BLOCS POLYOXYÉHTYLÈNE-POLYOXYPROPYLÈNE

(30) Priority: 04.06.2020 EP 20178260
(43) Date of publication of application: 12.04.2023
(73) Proprietor: Unilever IP Holdings B.V., 6708 WH Wageningen (NL); Unilever Global IP Limited, Wirral, Merseyside CH62 4ZD (GB)
(72) Inventor: CROTTY, Brian, Andrew, Trumbull, Connecticut 06611 (US); ELLISON, Matthew, David, Trumbull, Connecticut 06611 (US); MORIKIS, Thomas, Nikolaos, Trumbull, Connecticut 06611 (US); VELEZIS, Nicholas, Arthur, Trumbull, Connecticut 06611 (US)
(74) Representative: Unilever Patent Group
(86) International application number: PCT/EP2021/064414
(87) International publication number: WO 2021/244983

(56) References cited:
- EP-A1- 3 434 330
- CN-A- 109 172 387
- JP-A- 2004 203 794
- US-A- 3 740 421
- US-A1- 2007 053 853
- US-A1- 2011 008 266
- US-A1- 2011 224 299
- US-A1- 2019 343 907
- DATABASE GNPD [online] MINTEL; 6 February 2017 (2017-02-06), ANONYMOUS: "Chill Cooling and Hydrating Makeup Setting Spray", XP055739601, retrieved from www.gnpd.com Database accession no. 4595043
- DATABASE GNPD [online] MINTEL; 21 May 2019 (2019-05-21), ANONYMOUS: "Cool Astringent Tighten Up", XP055739563, retrieved from www.gnpd.com Database accession no. 6568221

## Description

### Field of the invention

The present invention is in the field of personal care compositions; in particular skin-benefit compositions that impart a cooling sensation on the skin. More particularly, the present invention relates to a personal care composition containing polyol; polyoxyethylene-polyoxypropylene block copolymer, a block copolymer solubilizer or a mixture thereof; a sensory modifier and water.

### Background of the invention

As the largest organ found on the human body, the skin is responsible for a vast array of bodily functions, including sensory reception, barrier protection and physiological regulation. However, it is frequently desirable to expedite the rate at which natural bodily functions occur. For instance, in hot, sunny, and/or humid climates, it is ideal to apply a product that is capable of soothing or cooling the skin after intense sun exposure to assist the skin's thermoregulation and barrier repair. It is to be noted that cooling personal care compositions are becoming increasingly popular, particularly in climates such as those noted above and in cultures where sunbathing is a norm. Cooling personal care compositions are also desirable for use when consumers, especially medical personnel, are wearing personal protective equipment.

Traditionally, a cooling effect of a personal care composition can be achieved by the inclusion of significant levels of ethanol whose volatile properties allow for a rapid evaporation upon application to the skin. Although effective, the cooling effect resulting from ethanol evaporation lasts for a relatively short period of time and can be drying for the skin. In addition, cooling agents such as menthol, camphor or ionic compounds, namely ammonium salts, are often used in topical personal care compositions to impart cooling sensations to the skin without physical cooling of the skin. However, use of such agents can often result in skin sensitization.

Therefore, the present inventors have recognized a need to develop a personal care composition that imparts a cooling effect while minimizing potential disadvantages such as those as previously noted. Thus, this invention is directed to a personal care composition having polyol; polyoxyethylene-polyoxypropylene block copolymer, a block copolymer solubilizer or a mixture thereof; a sensory modifier and water.

### Additional information

Efforts have been disclosed for creating cooling personal care compositions.

U.S. Patent No. 3740421 relates to polyoxyethylene-polyoxypropylene aqueous gels.

U.S. Patent No. 3867533 discloses an aqueous gel composition containing a water-insoluble organic ingredient using polyoxyethylene-polyoxypropylene block copolymers as gelling agents.

U.S. Patent No. 4407788 relates to a dentifrice with a liquid humectant vehicle in combination with siliceous polishing material in the presence of a resinous poly(ethylene oxide).

U.S. Patent Application Publication US 2002076423 relates to cooling cosmetic or dermatological formulations for post-sunbathing care comprising chitosan, lecithin, and a volatile substance.

U.S. Patent Application Publication US 20110224299 discloses a stabilized wax composition and uses thereof that may provide a cooling sensation when applied to the skin.

PCT application WO 2003/007909 relates to a cooling cosmetic or medicinal topical composition characterized by inclusion of methyl palmitate.

US2011008266 describes a cooling foam formulation comprising polyoxyethylene-polyoxypropylene block copolymers of the poloxamer type and the sensory modifier propane/ butane/isobutane
None of the additional information above describes the combination of poly (C₂-C₄ alkoxylate) block copolymers, a block copolymer solubilizer or a mixture thereof; polyol; a sensory modifier and water that results in a cooling effect when topically applied to the skin as claimed in the present invention.

### Summary of the invention

The present inventors have found a personal care composition that unexpectedly imparts a cooling effect when topically applied to the skin without needing to include an optional, traditional cooling agent.

Accordingly, in a first aspect, the invention relates to a cooling personal care composition comprising polyoxyethylene-polyoxypropylene block copolymer, a block copolymer solubilizer or a mixture thereof in combination with polyol, a sensory modifier and water as defined in the claims.

Also disclosed is a method of treating skin in need of treatment comprising the step of applying said cooling personal care composition onto skin in need of treatment.

All other aspects of the present invention will become more readily apparent upon considering the detailed description and examples which follow.

For the avoidance of doubt, the term "comprising" is meant not to be limiting to any stated elements but rather to encompass non-specified elements of major or minor functional importance. Therefore, the listed steps, elements or options need not be exhaustive. Whenever the words "including" or "having" are used, these terms are meant to be equivalent to "comprising" as defined above.

The term "skin" as used herein includes the skin on the face, neck, chest, back, arms, axilla, buttocks, hands, legs and scalp. Skin benefit agent, as used herein, is meant to include a component that (a) improves a facial or body characteristic after topical application like a skin characteristic, (b) benefits the same, or (c) both (a) and (b). The personal care composition is a composition for topical application and includes a lotion, gel, cream, serum, deodorant, antiperspirant, nail treatment and make-up. In an especially preferred embodiment, the personal care composition of this invention is a leave-on composition. The term "leave-on" as used with reference to compositions herein means a composition that is applied to or rubbed on the skin, and left thereon. "HLB" stands for the hydrophilic-lipophilic balance of a surfactant, used to measure the degree to which a surfactant is hydrophilic or lipophilic, and is widely used in the art. The term "derivative(s)" as used with reference to ingredients herein refers to functional group substitutions on the fatty acid backbone including hydroxy, alkoxy and C₁-C₄ alkyl substitutions. The term "solubilizer," as used herein, refers to an ingredient that helps to solubilize an ingredient otherwise insoluble in a medium. The use of "block copolymer" in the phrase "block copolymer solubilizer" is meant to clarify the characteristics of the solubilizer's chemical structure rather than clarify compounds the solubilizer is able to solubilize.

Except in the examples, or where otherwise explicitly indicated, all numbers in this description indicating amounts of ingredients are to be understood as modified by the word "about." All amounts are by total weight of the composition, unless otherwise specified.

### Detailed description of the invention

The polyol compounds comprised in the personal care composition of the present invention are polyhydric alcohol-type materials. Typical polyhydric alcohols include glycerol (i.e., glycerine or glycerin), propylene glycol, dipropylene glycol, polypropylene glycol, polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. The most preferred embodiment includes glycerin, butylene glycol, sorbitol or a mixture thereof. The amount of polyol employed may range anywhere from 0.5 to 3% by weight of the personal care composition.

A further component of the personal care composition according to the present invention is that of a copolymer surfactant, namely a poly(C₂-C₄ alkoxylate) block copolymer. The polymer comprises at least one oxyalkylene unit chosen from oxyethylene chains of formula (C₂H₄O)ₙ, in which n is chosen from integers ranging from 2 to 200, preferably, 5 to 150, oxypropylene chains of formula (C₃H₆O)_{n'}, in which n' is chosen from integers ranging from 2 to 100, preferably, 5 to 80, and random and block copolymers comprising chains chosen from oxyethylene chains of formula (C₂H₄O)ₙ, and oxypropylene chains of formula (C₃H₆O)_{n'}. The polyoxyethylene-polyoxypropylene block copolymer backbone chain terminates with a hydroxy terminal at one end and a hydride terminal at the other. The chemistry of the polyxoyethylene-polyoxypropylene block copolymer provides that the number of ethylene oxide groups is greater than the number of propylene oxide groups. In a preferred embodiment, the number of ethylene oxide groups to number of propylene oxide groups is at least in the ratio of 3:2. Furthermore, a polyxoyethylene-polyoxypropylene block copolymer suitable for use in the present invention is branched. In another preferred embodiment, the polyxoyethylene-polyoxypropylene block copolymer contains a branched propylene oxide group. The HLB value of the block copolymer is from 12 to 29, preferably, 14 to 28, and more preferably, 16 to 27. The number average (Mn) molecular weight of the block copolymer, as based on hydroxyl number, according to ASTM E222-17 Standard Test Methods, is in the range from 5,000 to 20,000, preferably, 7,000 to 19,000, most preferably, from 8,000 to 18,000. In a particularly preferred embodiment, Poloxamer 407, Poloxamer 338 or a mixture thereof is used in the invention. Poloxamer 407 is made commercially available from the supplier BASF under the Pluracare^{®} F127 trade name and Poloxamer 308 is commercially available from BASF under the Pluracare^{®} F108 trade name. Total concentration of the polyxoyethylene-polyoxypropylene block copolymer may range from 0.2 to 5% by weight of the personal care composition.

It is within the scope of the invention to employ a block copolymer solubilizer in place of or in combination with the copolymer surfactant described above. In an embodiment of the present invention, the block copolymer solubilizer material is a polyethylene glycol-polypropylene glycol block copolymer reaction product of ethylene oxide-propylene oxide polymerization. The HLB value of the block copolymer solubilizer are in the range from 8 to 17, preferably, 9 to 15, and more preferably, 10 to 13. The number average molecular weight of the block copolymer solubilizer, as based on hydroxyl number according to ASTM E222-17 Standard Test Methods, is in the range from 5,000 to 20,000, preferably, 7,000 to 19,000, and most preferably, from 8,000 to 18,000. A block copolymer solubilizer suitable for use in the invention is made commercially available as PEG/PPG-116/66 Copolymer under the trade name Pluracare^{®} L1220 by BASF. Total concentration of the block copolymer solubilizer may range from 0.2 to 5% by weight of the personal care composition.

The cooling personal care composition of the present invention comprises a sensory modifier.

Sensory modifiers include linear or branched C₉-C₂₂ alkanes; linear or branched C₅-C₁₈ fatty acid esters; silicones or a mixture thereof. Derivatives of such sensory modifiers may also be suitable for use. The sensory modifier may be present in the present invention from 0.3 to 2%.

Alkanes (C₉-C₂₂) of the linear or branched variants may be used in the invention. Thus, it is within the scope of the present invention to include hydrocarbons. Suitable hydrocarbons may include mineral oil and polyalpha-olefins. Examples of preferred volatile hydrocarbons include polydecanes such as undecane, tridecane, isodecane, isohexadecane and isododecane and the C₇-C₈ through C₁₂-C₁₅ isoparaffins as well as any mixtures thereof.

Fatty acid esters (C₅-C₁₈) are also suitable for use in the personal care composition of the present invention. Illustrative but non-limiting examples include fatty acid esters derived from acid precursors such as pivalic acid (e.g. isodecyl neopentoate), isononanoic acid (e.g. ethylhexyl isononanoate, isononyl isononanoate, isodecyl isononanoate and PEG-2 diisononanoate), 2-ethylhexanoic acid (e.g. PEG-2 diethylhexanoate, isodecyl ethylhexanoate), decanoic acid (e.g. hexyl decanoate), caprylic acid (e.g. coco-caprylate, propylheptyl caprylate, caprylyl caprylate/caprate), isostearic acid (e.g. methylheptyl isostearate), heptanoic acid (e.g. neopentyl glycol diheptanoate), myristic acid (e.g. isopropyl myristate), palmitic acid (e.g. isopropyl palmitate) or mixtures thereof.

It is also within the scope of the invention to employ ingredients from the group known as triglycerides. Illustrative but non-limiting examples are sunflower seed oil, cotton oil, canola oil, grapeseed oil, soybean oil, castor oil, borage oil, olive oil, shea butter, jojoba oil and mixtures thereof. Mono- and di-glycerides may also be useful. Illustrative of these categories are glyceryl monostearate and glyceryl distearate. Amounts of triglycerides may range from 0.01 to 10%, preferably, from 0.1 to 8% by weight of the composition.

Silicones suitable for use may be categorized into the volatile and nonvolatile variety. Amounts may range, for example, from 0.01 to 25%, more preferably, from 0.1 to 20% by weight of the composition. The term "volatile" as used herein refers to those materials which have a measurable vapor pressure at 25°C. Volatile silicone oils are preferably chosen from cyclic (cyclomethicone) or linear polydimethylsiloxanes containing from 3 to 9, more preferably, from 4 to 5, silicon atoms.

Nonvolatile silicones useful in this composition include polyalkyl siloxanes, polyalkylaryl siloxanes and polyether siloxane copolymers. The essentially nonvolatile polyalkyl siloxanes useful herein include, for example, polydimethyl siloxanes with viscosities from about 5 x 10⁻⁶ to 0.1 m²/s at 25°C. Emulsifying and non-emulsifying silicone elastomers are also suitable for use in the personal care composition of this invention.

Personal care composition of the present invention may also comprise a cosmetically acceptable vehicle. This cosmetically acceptable vehicle may be aqueous or an emulsion. Oily carriers in the presence of water and an emulsifier will form emulsion systems as carriers. Preferably, the compositions are aqueous or an emulsion, especially oil-in-water emulsion. The personal care compositions ordinarily will be in but are not limited to a gelled form. Suitable carriers employed in the invention include water, sea water, rosewater (e.g. *Rosa Damascena* flower Water), tea (e.g. *Camellia Sinensis* Leaf Water), *Aloe Barbadensis* (aloe vera) leaf juice, witch hazel (e.g. *Hamamelis Virginiana* extract), lavender water (e.g. *Lavendula Angustifolia* flower water), grape water (e.g. *Vitis Vinifera* fruit water and *Vitis Vinifera* juice). In a particularly preferred embodiment, the carrier will be water and the final personal care composition is an aqueous gel. The cosmetically acceptable vehicle will usually form from 70% to 98%, preferably, from 75 to 97%, and more preferably, 80 to 95% by weight of the personal care composition, and can, in the absence of other cosmetic adjuncts, form the balance of the composition. In a preferred embodiment, the vehicle is at least 80% water, by weight of the cosmetically acceptable vehicle.

In the present invention, the composition comprises a carrier which is water, in which the personal care composition is an aqueous gel, and the carrier forms from 80 to 98% by weight of the personal care composition.

Fragrances, thickening agents, skin benefit agents, fixatives and abrasives may optionally be included in personal care compositions of the present invention. Skin benefit agents suitable for use in this invention are meant to include but not be limited to opacifiers, colorants, humectants, emollients, occlusive agents, plant extracts, optical agents, cooling agents, skin lightening agents, anti-inflammatory agents, anti-acne agents, sunscreens, photostabilizers, wrinkle reducing agents, desquamation promoters, exfoliating agents, mixtures thereof or the like. Each of these substances, when used, may range from 0.05 to 5%, preferably, between 0.1 and 3% by weight of the total weight of the personal care composition.

The personal care composition may optionally comprise one or more thickening agents, preferably, from 0.05 to 10%, more preferably, from 0.1 to 5%, and even more preferably, from 0.25 to 4%, by weight of the personal care composition. Useful thickeners include polysaccharides, which comprise of starches, natural/synthetic gums and cellulosics. Suitable starches include tapioca starch, cornstarch, potato starch, aluminum starch octenylsuccinate and sodium hydroxypropyl starch phosphate. Suitable gums include xanthan, sclerotium, pectin, karaya, arabic, agar, guar, carrageenan, alginate and combinations thereof. Suitable cellulosics include hydroxypropyl cellulose, hydroxypropyl methylcellulose, ethylcellulose and sodium carboxy methylcellulose. Synthetic polymers functioning as thickening agents are also suitable for use, including polyacrylamides, Carbomers, taurate copolymers, and acrylate-based polymers.

Fatty acids may also be useful ingredients to incorporate for use in the present invention. The term "fatty" refers to carbon chain lengths ranging from 10 to 30 carbon atoms. Illustrative of this category are pelargonic, lauric, myristic, palmitic, stearic, isostearic, hydroxystearic, oleic, linoleic, ricinoleic, arachidic, behenic, erucic acids and combinations thereof. When used, from 0.01 to 5% by weight fatty acid is present in the composition.

Waxes and wax esters are also suitable for use in the present invention. These waxes include petroleum-derived waxes (e.g. paraffin wax, petrolatum, microcrystalline wax), animal-derived waxes or those from animal byproducts (e.g. beeswax, spermaceti wax, Chinese wax, wool wax, shellac wax), plant-derived waxes (e.g. tribehenin wax, carnauba wax, candelilla wax, bayberry wax, jojoba wax, orange wax, rice bran wax, sunflower wax, castor wax, soy wax), mineral waxes (e.g. montan wax, ceresin wax, ozokerite) and synthetic derivatives of natural waxes. The amount of wax and wax esters, if employed, may range from 0.01 to 10%, preferably, from 0.1 to 8% by weight of the composition.

Emulsifiers are optionally present in the personal care composition of the present invention. Emulsifiers are often used when the desired cosmetically acceptable vehicle is an emulsion. Emulsifiers suitable for use in the present invention will have an HLB from 2.5 to 17. The HLB value of the emulsifier used when water-continuous emulsions are desired will be 8 to 17, preferably, from 8.5 to 15, and most preferably, from 9 to 14, including all ranges subsumed therein.

Total concentration of the emulsifier, when used, may range from 0.1 to 20%, preferably, from 1 to 10%, and most preferably, from 1 to 8% by weight of the composition, including all ranges subsumed therein. The emulsifier may be selected from the group consisting of anionic, nonionic, cationic and amphoteric surfactants. Particularly preferred nonionic surfactants are those with a C₁₀-C₂₀ fatty alcohol or acid hydrophobe condensed with from about 2 to about 100 moles of ethylene oxide or propylene oxide per mole of hydrophobe; C₂-C₁₀ alkyl phenols condensed with from 2 to 20 moles of alkylene oxide; mono- and di-fatty acid esters of ethylene glycol; fatty acid monoglyceride; sorbitan, mono- and di- C₈-C₂₀ fatty acids; and polyoxyethylene sorbitan as well as combinations thereof. Alkyl polyglycosides and saccharide fatty amides (e.g. methyl gluconamides) are also suitable nonionic emulsifiers.

Preferred anionic emulsifiers include alkyl ether sulfate and sulfonates, alkyl sulfates and sulfonates, alkylbenzene sulfonates, alkyl and dialkyl sulfosuccinates, C₈-C₂₀ acyl isethionates, C₈-C₂₀ acyl methyl isethionates, C₈-C₂₀ acyl methyl taurates, C₈-C₂₀ alkyl ether phosphates, alkyl ether carboxylates or combinations thereof.

Cationic emulsifiers that may be used include, for example, palmitamidopropyltrimonium chloride, distearyldimonium chloride, diester quaternary ammonium compounds (e.g. distearoylethyl dimonium chloride) or mixtures thereof.

Useful amphoteric emulsifiers include cocoamidopropyl betaine, C₁₂-C₂₀ trialkyl betaines, sodium lauroamphoacetate, and sodium laurodiamphoacetate or a mixture thereof.

Other generally preferred emulsifiers include glyceryl stearate, glycol stearate, stearamide AMP, PEG-100 stearate, as well as emulsifying/thickening additives like hydroxyethylacrylate/sodium acryloyldimethyl taurates copolymer/squalane and mixtures thereof.

Personal care compositions of the present invention may include vitamins as the desired skin benefit agent, including derivatives thereof such as vitamin alkyl esters. Illustrative vitamins are vitamin A, vitamin B₂, vitamin B₃ (niacinamide), vitamin B₅ (panthenol), vitamin B₆, vitamin C, vitamin E, vitamin K, folic Acid and biotin. Derivatives of the vitamins may also be employed. For instance, vitamin C derivatives include ascorbyl tetraisopalmitate, magnesium ascorbyl phosphate and ascorbyl glucoside. Derivatives of vitamin E include tocopheryl acetate, tocopheryl palmitate and tocopheryl linoleate. Derivatives of vitamin B₃ include nicotinic acid. If employed, total amount of vitamins when present in personal care compositions according to the present invention may range from 0.001 to 10%, preferably, from 0.01 to 5%, optimally, from 0.05 to 2.5% by weight of the personal care composition.

Additional optional skin benefit agents suitable for use in this invention include minerals and skin nutrients such as milk; magnesium, calcium, copper, zinc and other metallic components; retinoic acid and its derivatives (e.g., cis and trans); retinal; retinol; retinyl esters such as retinyl acetate, retinyl palmitate, and retinyl propionate; alpha hydroxy acids, beta hydroxy acids, e.g. salicylic acid and derivatives thereof (such as 5-octanoyl salicylic acid, heptyloxy 4 salicylic acid, and 4-methoxy salicylic acid); resorcinol derivatives (particularly 4-substituted resorcinol derivatives, including 4-ethyl resorcinol, 4-isopropyl resorcinol, 4-hexyl resorcinol, 4-cyclopentyl resorcinol, 4-cyclohexyl resorcinol and acylated forms thereof); resveratrol; saccharide isomerate; ceramides (e.g. Ceramide 1, Ceramide 3, Ceramide 3B and Ceramide 6) and pseudoceramides; allantoin; pentaerythrityl tetra-di-t-butyl hydroxyhydrocinnamate; pyroglutamic acid (PCA) salt derivatives including zinc PCA and sodium PCA; skin benefit acids such as 12-hydroxystearic acid, petroselinic acid, conjugated linoleic acid, octadecanoic acid, hyaluronic acid and its salt derivatives; mixtures thereof or the like. While the cooling capability of the system is already sufficient, cooling agents may also be optionally added, including menthol or derivatives thereof, camphor or derivatives thereof and ionic compounds such as ammonium salts, to increase the perception of cooling on the skin. Ethanol may also optionally be added for use in cooling of the skin. Such skin benefit agents, when used, collectively make up from 0.001 to 12% by weight of the personal care composition.

A wide selection of botanical extracts may optionally be included in personal care compositions of this invention. The extracts may either be soluble in water or oil, carried in a solvent that is hydrophilic or hydrophobic, respectively. In the preferred embodiment, water or ethanol are the extract solvents. Illustrative examples include those extracted from green tea, yarrow, chamomile, licorice, aloe vera, citrus unshui, willow bark, alfalfa, algae, witch hazel, sage, thyme and rosemary, as well as oils such as those derived from sea buckthorn, moringa, argan, avocado, calendula, algal and marula. Other essential oils that impart a cooling effect can optionally be used, including those extracted from peppermint, spearmint, lavender, eucalyptus, sandalwood and vetiver. Soy extracts may be used and especially when it is desirable to include retinol. These extracts, if used, may be included from 0.001 to 12% by total weight of the personal care composition.

Another optional additive suitable for use includes hemp oil with 2.5 to 25% by weight cannabigerol and/or cannabidiol at from 0.5 to 10 percent by weight. When used, such oil makes up from 0.0001 to 12% by weight of the composition, and preferably, from 0.01 to 5% by weight of the personal care composition, including all ranges subsumed therein.

Traditional buffers or pH modifiers are also suitable for inclusion to the personal care compositions of this invention. These include common additives such as sodium hydroxide, potassium hydroxide, hydrochloric acid, citric acid, triethanolamine, tetrahydroxypropyl ethylenediamine and aminomethyl propanol. In a preferred embodiment, the pH of the personal care composition of the present invention is from 3 to 8, and more preferably, from 3.25 to 7.75, and most preferably, from 4 to 7.5. Viscosity of the personal care composition of this invention is preferably from about 1,000 to about 120,000 cps, and most preferably, from about 5,000 to 80,000 cps, taken under conditions of 25°C and a shear rate of 1s⁻¹ with a strain controlled parallel plate rheometer made commercially available from suppliers like T.A. Instruments under the Discovery name. Alternatively, viscosity can also be measured using a Brookfield Viscometer (speed at 20 rpm, spindle 6, helipath off, for 30 seconds at 25°C).

Preservatives can be incorporated into the compositions of this invention as desired to protect against the growth of potentially harmful microorganisms. Personal care chemists are familiar with appropriate preservatives and routinely choose them to satisfy preservative tests and product stability tests. Preservative systems should be selected having regard for the use of the composition and possible incompatibilities between the preservatives and other ingredients in the formulation. Exemplary examples of preservatives for compositions of this invention include, without limitations, iodopropynyl butyl carbamate (IPBC), phenoxyethanol, ethylhexylglycerin, methyl paraben, propyl paraben, imidazolidinyl urea, sodium dehydroacetate, sodium benzoate, benzoic acid, alkyl esters of para-hydroxybenzoic acid, benzyl alcohol, hydroxyacetophenone, DMDM hydantoin derivatives, climbazole, propionate salts, and a variety of quaternary ammonium compounds. When used, preservatives are preferably employed in amounts ranging from 0.01 to 2% by weight of the composition, including all ranges subsumed therein.

The personal care composition of this invention is a composition suitable for topical application to human skin, particularly leave-on products, to deliver a skin benefit. Preferably, the term encompasses a gel, and particularly a moisturizer rather than a make-up product. Most preferred are leave-on compositions.

Many types of packaging can be used to store and deliver the end-use composition of the present invention. The selection of packaging is dependent upon the personal care end-use and the viscosity of the composition itself. As an example, leave-on lotions and creams for skin typically employ plastic containers with an opening at a dispense end covered by an appropriate closure. Conventional closures include flip-top hinged lids and screw-caps. In general, patches, bottles, tubes, roller-ball applicators, squeeze containers or lidded jars are preferred.

Cooling effect of a personal care composition on skin can be measured with an IR-thermometer, such as the Fluke 62 Mini IR thermometer made commercially available by Fluke Corporation. Initial skin temperature of a panelist's forearm is measured using the IR-thermometer at 25°C and atmospheric conditions and recorded. Samples are weighed using a conventional scale and weight boat and 0.5 grams of each sample is measured. Each sample is individually spread evenly along the panelist's forearm for 30 seconds. The IR-thermometer is used to scan the total area of product applied and the initial drop in skin temperature post-application is recorded. Temperature measurements are subsequently taken at designated intervals (e.g. every 30 seconds) until an absolute minimum temperature has been established. The difference between the initial temperature measurement and the absolute minimum temperature measurement is denoted as ΔT_{avg}. ΔT_{avg} values obtained from the application of sample compositions made according to the present invention are compared to ΔT_{avg} values of the anchor composition.

The invention will now be described below in the context of specific non-limiting examples to facilitate a greater understanding of the present invention. One of ordinary skill in the art will recognize that variations of the present invention that differ from the examples given may be practiced without deviating from the teachings of the present invention.

### Examples

All samples were made by mixing the mentioned ingredients under conditions of moderate sheer, about 25°C to about 75°C, and atmospheric pressure.

All samples in the Examples below were compared to a control composition, hereafter referred to as the anchor, made commercially available by Aveeno^{®} as Aveeno^{®} Active Naturals Skin Relief Moisturizing Cream with Cooling Action. As it pertains to the present invention, the anchor used herein contains a polyol (i.e. glycerin), a sensory modifier (i.e. dimethicone) and menthol.

### Example 1: Full basic formulation according to the present invention

| **Ingredients** | **wt.%** |
|---|---|
| Water | Balance |
| Preservative | 0.25 |
| Colorant (0.0075%) | 0.38 |
| Glycerin | 1.0 |
| Opacifier | 0.50 |
| Poloxamer | 0.50 |
| Acrylates/C10-30 Alkyl Acrylate Crosspolymer | 0.50 |
| Petrolatum | 0.01 |
| Dimethicone | 0.50 |
| Cyclopentasiloxane | 0.50 |
| Vitamin | 0.01 |
| Preservative | 0.60 |
| pH adjuster (85%) | 0.88 |
| Fragrance | 0.40 |
| Skin benefit agents | 1.0 |
| Menthol | 0.10 |

This full formulation shows the basic formulation used in experiments below, upon which modifications were made. This full formulation is hereafter referred to as "FF."

### Example 2: Impact of presence of menthol in FF on cooling effect

| Sample | Poloxamer 407 (wt. %) | Cyclopentasiloxane (wt. %) | Glycerin (wt. %) | ΔT_{avg} (°C) |
|---|---|---|---|---|
| Anchor | 0.0 | - | - | -3.8 |
| FF with 0% menthol | 0.5 | 0.5 | 1.0 | -5.4 |
| FF | 0.5 | 0.5 | 1.0 | -5.5 |

The results demonstrate that the full formulation made according to the present invention unexpectedly results in a greater average drop in temperature on the surface of the skin (ΔT_{avg}) than the anchor sample by nearly 2°C. It can also be seen that full formulation with or without the inclusion of menthol will achieve the same result with negligible impact to average drop in temperature.

### Example 3: Impact of different copolymer surfactants and/or block copolymer solubilizer on cooling effect

| Sample | Pluracare^{®} (wt. %) | Cyclopentasiloxane (wt. %) | Glycerin (wt. %) | ΔT_{avg} (°C) |
|---|---|---|---|---|
| Anchor | 0.0 | - | - | -3.8 |
| FF with Pluracare^{®} F127 | 0.5 | 0.5 | 1.0 | -5.5 |
| FF with Pluracare^{®} F108 | 0.5 | 0.5 | 1.0 | -5.3 |
| FF with Pluracare^{®} L1220 | 0.5 | 0.5 | 1.0 | -5.7 |

Pluracare^{®} is the trade name collection for surfactants comprising ethylene oxide and propylene oxide units or reaction products of such units that is made commercially available from BASF. The chemical names for Pluracare^{®} F127 is, Pluracare^{®} F108, and Pluracare^{®} L1220 are Poloxamer 407, Poloxamer 338 and PEG/PPG-116/66 Copolymer, respectively.

The results illustrate that replacing a copolymer surfactant with other copolymer surfactants or block copolymer solubilizers can result in similar ΔT_{avg} values on the surface of the skin.

### Example 4: Impact of varying sensory modifier levels on cooling effect

| Sample | Poloxamer 407 (wt. %) | Cyclopentasiloxane (wt. %) | Glycerin (wt. %) | ΔT_{avg} (°C) |
|---|---|---|---|---|
| Anchor | 0.0 | - | - | -3.8 |
| FF | 0.5 | 0.5 | 1.0 | -5.5 |
| FF with 0.1% cyclopentasiloxane | 0.5 | 0.1 | 1.25 | -5.3 |
| FF with 1.0% cyclopentasiloxane | 0.5 | 1.0 | 1.25 | -5.0 |
| FF with 1.5% cyclopentasiloxane | 0.5 | 1.5 | 1.25 | -4.4 |

All sample compositions in Example 4 were made using Poloxamer 407 and demonstrate the effect of changing sensory modifier inclusion levels (i.e. cyclopentasiloxane) on cooling. Thus, one of ordinary skill in the art can see that increasingly levels of sensory modifier can result in similar dips in ΔT_{avg}.

### Example 5: Impact of different sensory modifiers on cooling effect

| Sample | Poloxamer 407 (wt. %) | Sensory modifer (wt. %) | Glycerin (wt. %) | ΔT_{avg} (°C) |
|---|---|---|---|---|
| Anchor | 0.0 | - | - | -3.8 |
| FF | 0.5 | 0.5 | 1.0 | -5.5 |
| FF with 0.5% C₁₂₋₁₇ alkane | 0.5 | 0.5 | 1.25 | -5.4 |
| FF with 0.5% C₁₄₋₂₂ alkane | 0.5 | 0.5 | 1.25 | -5.4 |
| FF with 0.5% Dimethicone 5 cSt | 0.5 | 0.5 | 1.25 | -5.9 |
| FF with 0.5% isopropyl myristate | 0.5 | 0.5 | 1.25 | -6.8 |

All sample compositions in Example 5 were made using Poloxamer 407 at 0.5%. Modifications upon the full formulation made according to the composition in Example 1 all comprise consistent levels of glycerin at 1.25%. Unexpectedly, it can be seen that replacing a particular sensory modifier for another can yield either a similar or even more significant average drop in temperature on the surface of the skin when compared to the full formulation.

In this particular example, the C₁₂₋₁₇ alkane employed is made commercially available by Sonneborn under the tradename Iris^{®}, the C₁₄₋₂₂ alkane employed is made commercially available by Sonneborn under the tradename Lilac^{®}, the Dimethicone 5 cSt employed is made commercially available by Dow Corning under the tradename XIAMETER^{™} PMX-200 Silicone Fluid 5 cSt.

### Example 6: Impact of different polyols on cooling effect

| Sample | Poloxamer 407 (wt. %) | Cyclopentasiloxane (wt. %) | Polyol (wt. %) | ΔT_{avg} (°C) |
|---|---|---|---|---|
| Anchor | 0.0 | - | - | -3.8 |
| FF | 0.5 | 0.5 | 1.0 | -5.5 |
| FF with 1.25% butylene glycol | 0.5 | 0.5 | 1.25 | -7.0 |
| FF with 1.25% pentylene glycol | 0.5 | 0.5 | 1.25 | -4.4 |
| FF with 1.25% sorbitol | 0.5 | 0.5 | 1.25 | -5.7 |

All sample compositions in Example 6 were made using Poloxamer 407 at 0.5% and cyclopentasiloxane at 0.5%. The results surprisingly illustrate that the use of different polyols can yield either a similar or greater average drop in temperature on the surface of the skin when compared to the full formulation. In the sample prepared with pentylene glycol, though exhibiting a lower dip in ΔT_{avg} compared to FF, it still demonstrates approximately a 1°C additional decrease on top of the anchor's ΔT_{avg}.

### Example 7: Impact of polyol and/or copolymer surfactants on cooling effect

| Sample | Poloxamer 407 (wt. %) | Cyclopentasiloxane (wt. %) | Glycerin (wt. %) | ΔT_{avg} (°C) |
|---|---|---|---|---|
| Anchor | 0.0 | - | - | -3.8 |
| FF | 0.5 | 0.5 | 1.0 | -5.5 |
| FF with 6% glycerin | 0.5 | 0.5 | 6.0 | -4.5 |
| FF with 0.1% Poloxamer + 0.5% glycerin | 0.1 | 0.5 | 0.50 | -4.7 |
| FF with 0.1% Poloxamer + 2% glycerin | 0.1 | 0.5 | 2.0 | -5.2 |
| FF with 0.1% Poloxamer + 10% glycerin | 0.1 | 0.5 | 10 | -3.5 |
| FF with 1% Poloxamer + 0.5% glycerin | 1.0 | 0.5 | 0.50 | -5.2 |
| FF with 1% Poloxamer + 2% glycerin | 1.0 | 0.5 | 2.0 | -5.8 |
| FF with 1% Poloxamer + 10% glycerin | 1.0 | 0.5 | 10 | -3.9 |
| FF with 3% Poloxamer + 1.25% glycerin | 3.0 | 0.5 | 1.3 | -6.0 |
| FF with 3% Poloxamer + 6% glycerin | 3.0 | 0.5 | 6.0 | -5.2 |
| FF with 5% Poloxamer + 0.5% glycerin | 5.0 | 0.5 | 0.50 | -5.5 |
| FF with 5% Poloxamer + 2% glycerin | 5.0 | 0.5 | 2.0 | -4.8 |
| FF with 5% Poloxamer + 10% glycerin | 5.0 | 0.5 | 10 | -4.0 |

All sample compositions in Example 7 were made using Poloxamer 407. By varying both inclusion levels of glycerin and the copolymer surfactant, the results of this Example show that there is some fluctuation. It is noted that this fluctuation is not observed to be significant until the inclusion level of glycerin in the composition is at least 10%. However, even after this drop in effectiveness is observed, the cooling effect achieved is still comparable to that of the anchor formulation across all samples having 10% glycerin.

## Claims

1. A personal care composition comprising:
(a) from 0.5 to 3% by weight of the personal care composition of a polyol, which polyol comprises glycerin, butylene glycol, sorbitol, or a mixture thereof;
(b) from 0.2 to 5% by weight of the personal care composition of a polyoxyethylene-polyoxypropylene block copolymer, block copolymer solubilizer, or a mixture thereof;
(c) from 0.3 to 2% by weight of a personal care composition of a sensory modifier, which sensory modifier comprises a linear or branched C₉-C₂₁ alkane, linear or branched C₅-C₁₈ fatty acid ester, silicone, or a mixture thereof; and
(d) a carrier which is water, in which the personal care composition is an aqueous gel, and the carrier forms from 80 to 98% by weight of the personal care composition.

2. The personal care composition according to Claim 1, wherein the polyoxyethylene-polyoxypropylene block copolymer comprises at least one oxyalkylene unit chosen from oxyethylene chains of formula (C₂H₄O)ₙ, where n is chosen from integers ranging from 2 to 200, at least oxypropylene chains of formula (C₃H₆O)_{n'}, where n' is chosen from integers ranging from 2 to 100, and random and block copolymers comprising chains chosen from oxyethylene chains of formula (C₂H₄O)ₙ, and oxypropylene chains of formula (C₃H₆O)_{n'}.

3. The personal care composition according to Claim 2, wherein the number of ethylene oxide units to number of propylene oxide units is in the ratio of at least 3:2.

4. The personal care composition according to any of the preceding claims, wherein the polyoxyethylene-polyoxypropylene block copolymer has an HLB value of 12 to 29.

5. The personal care composition according to any of the preceding claims, wherein the polyoxyethylene-polyoxypropylene block copolymer has an HLB value of 14 to 28.

6. The personal care composition according to any of the preceding claims, wherein the block copolymer solubilizer has an HLB value of 8 to 17.

7. The personal care composition according to any of the preceding claims, wherein the block copolymer solubilizer has an HLB value of 9 to 15.

8. The personal care composition according to Claim 1, wherein the polyoxyethylene-polyoxypropylene block copolymer or block copolymer solubilizer has a number average molecular weight of 5,000 to 20,000.

9. The personal care composition according to Claim 1, wherein the polyoxyethylene-polyoxypropylene block copolymer or block copolymer solubilizer has a number average molecular weight of 7,000 to 19,000.

10. Use of the composition of Claim 1 to cool skin after application.

11. The personal care composition according to Claim 1, wherein the composition further comprises menthol, camphor, ethanol, a cooling agent, or a mixture thereof.

## Patentansprüche

1. Körperpflegezusammensetzung, umfassend:
(a) 0,5 bis 3 Gew.-% der Körperpflegezusammensetzung eines Polyols, wobei das Polyol Glycerin, Butylenglykol, Sorbit oder eine Mischung davon umfasst;
(b) 0,2 bis 5 Gew.-% der Körperpflegezusammensetzung eines Polyoxyethylen-Polyoxypropylen-Blockcopolymers, eines Blockcopolymer-Lösungsvermittlers oder einer Mischung davon;
(c) 0,3 bis 2 Gew.-% der Körperpflegezusammensetzung eines sensorischen Modifikators, wobei der sensorische Modifikator ein lineares oder verzweigtes C₉-O₂₁-Alkan, einen linearen oder verzweigten C₅-O₁₈-Fettsäureester, Silikon oder eine Mischung davon umfasst; und
(d) einen Träger, der Wasser ist,
wobei die Körperpflegezusammensetzung ein wässriges Gel ist und der Träger 80 bis 98 Gew.-% der Körperpflegezusammensetzung ausmacht.

2. Körperpflegezusammensetzung nach Anspruch 1, wobei das Polyoxyethylen-Polyoxypropylen-Blockcopolymer mindestens eine Oxyalkyleneinheit umfasst, die ausgewählt ist unter Oxyethylenketten der Formel (C₂H₄O)ₙ, wobei n unter ganzen Zahlen im Bereich von 2 bis 200 ausgewählt ist, mindestens Oxypropylenketten der Formel (C₃H₆O)_{n'}, wobei n' unter ganzen Zahlen im Bereich von 2 bis 100 ausgewählt ist, und statistischen und Blockcopolymeren, umfassend Ketten, die unter Oxyethylenketten der Formel (C₂H₄O)ₙ und Oxypropylenketten der Formel (C₃H₆O)_{n'} ausgewählt sind.

3. Körperpflegezusammensetzung nach Anspruch 2, wobei das Verhältnis der Anzahl der Ethylenoxideinheiten zur Anzahl der Propylenoxideinheiten mindestens 3:2 beträgt.

4. Körperpflegezusammensetzung nach einem der vorhergehenden Ansprüche, wobei das Polyoxyethylen-Polyoxypropylen-Blockcopolymer einen HLB-Wert von 12 bis 29 aufweist.

5. Körperpflegezusammensetzung nach einem der vorhergehenden Ansprüche, wobei das Polyoxyethylen-Polyoxypropylen-Blockcopolymer einen HLB-Wert von 14 bis 28 aufweist.

6. Körperpflegezusammensetzung nach einem der vorhergehenden Ansprüche, wobei der Blockcopolymer-Lösungsvermittler einen HLB-Wert von 8 bis 17 aufweist.

7. Körperpflegezusammensetzung nach einem der vorhergehenden Ansprüche, wobei der Blockcopolymer-Lösungsvermittler einen HLB-Wert von 9 bis 15 aufweist.

8. Körperpflegezusammensetzung nach Anspruch 1, wobei das Polyoxyethylen-Polyoxypropylen-Blockcopolymer oder der Blockcopolymer-Lösungsvermittler ein zahlenmittleres Molekulargewicht von 5.000 bis 20.000 aufweist.

9. Körperpflegezusammensetzung nach Anspruch 1, wobei das Polyoxyethylen-Polyoxypropylen-Blockcopolymer oder der Blockcopolymer-Lösungsvermittler ein zahlenmittleres Molekulargewicht von 7.000 bis 19.000 aufweist.

10. Verwendung der Zusammensetzung nach Anspruch 1, um nach dem Auftragen zur Hautkühlung zu führen.

11. Körperpflegezusammensetzung nach Anspruch 1, wobei die Zusammensetzung ferner Menthol, Kampfer, Ethanol, ein Kühlmittel oder eine Mischung davon umfasst.

## Revendications

1. Composition de soins personnels comprenant :
(a) de 0,5 à 3 % en poids de la composition de soins personnels d'un polyol, ledit polyol comprenant la glycérine, le butylène glycol, le sorbitol, ou un mélange de ceux-ci ;
(b) de 0,2 à 5 % en poids de la composition de soins personnels d'un copolymère séquencé de polyoxyéthylène - polyoxypropylène, solubilisant de copolymère séquencé, ou d'un mélange de ceux-ci ;
(c) de 0,3 à 2 % en poids de la composition de soins personnels d'un modificateur sensoriel, ledit modificateur sensoriel comprenant un alcane en C₉ à C₂₁ linéaire ou ramifié, un ester d'acide gras en C₅ à C₁₈ linéaire ou ramifié, une silicone ou un mélange de ceux-ci ; et
(d) un support qui est l'eau, dans laquelle la composition de soins personnels est un gel aqueux et le support forme de 80 à 98 % en poids de la composition de soins personnels.

2. Composition de soins personnels selon la revendication 1, dans laquelle le copolymère séquencé de polyoxyéthylène - polyoxypropylène comprend au moins un motif oxyalkylène choisi parmi des chaînes oxyéthylène de formule (C₂H₄O)ₙ, où n est choisi parmi des entiers allant de 2 à 200, au moins des chaînes oxypropylène de formule (C₃H₆O)_{n'}, où n' est choisi parmi des entiers allant de 2 à 100, et les copolymères aléatoires et séquencés comprenant des chaînes choisies parmi des chaînes oxyéthylène de formule (C₂H₄O)ₙ et des chaînes oxypropylène de formule (C₃H₆O)_{n'}.

3. Composition de soins personnels selon la revendication 2, dans laquelle le nombre de motifs oxyde d'éthylène sur le nombre de motifs oxyde de propylène est dans le rapport d'au moins 3/2.

4. Composition de soins personnels selon l'une quelconque des revendications précédentes, dans laquelle le copolymère séquencé de polyoxyéthylène - polyoxypropylène a une valeur HLB de 12 à 29.

5. Composition de soins personnels selon l'une quelconque des revendications précédentes, dans laquelle le copolymère séquencé de polyoxyéthylène - polyoxypropylène a une valeur HLB de 14 à 28.

6. Composition de soins personnels selon l'une quelconque des revendications précédentes, dans laquelle le solubilisant de copolymère séquencé a une valeur HLB de 8 à 17.

7. Composition de soins personnels selon l'une quelconque des revendications précédentes, dans laquelle le solubilisant de copolymère séquencé a une valeur HLB de 9 à 15.

8. Composition de soins personnels selon la revendication 1, dans laquelle le copolymère séquencé de polyoxyéthylène - polyoxypropylène ou le solubilisant de copolymère séquencé a un poids moléculaire moyen en nombre de 5000 à 20 000.

9. Composition de soins personnels selon la revendication 1, dans laquelle le copolymère séquencé de polyoxyéthylène - polyoxypropylène ou le solubilisant de copolymère séquencé a un poids moléculaire moyen en nombre de 7000 à 19 000.

10. Utilisation de la composition selon la revendication 1 pour rafraîchir la peau après application.

11. Composition de soins personnels selon la revendication 1, la composition comprenant en outre du menthol, du camphre, de l'éthanol, un agent rafraîchissant, ou un mélange de ceux-ci.
